# EUROPEAN PATENT SPECIFICATION

(11) **EP 4 619 108 B1**
(45) Date of publication and mention of the grant of the patent: **02.12.2026**
(21) Application number: 23798372.1
(22) Date of filing: 25.10.2023
(51) Int. Cl.: A61Q 11/00, A61K 8/04, A61K 8/25, A61K 8/44, A61K 8/73

(54) **ORAL CARE COMPOSITION**
MUNDPFLEGEZUSAMMENSETZUNG
COMPOSITION DE SOIN BUCCAL

(30) Priority: 17.11.2022 WO PCT/CN2022/132442; 09.12.2022 EP 22212380
(43) Date of publication of application: 24.09.2025
(73) Proprietor: Unilever IP Holdings B.V., 6708 WH Wageningen (NL); Unilever Global IP Limited, Wirral, Merseyside CH62 4ZD (GB)
(72) Inventor: GAO, Long, 6708 WH Wageningen (NL); LIU, Weining, 6708 WH Wageningen (NL); WANG, Xiaohong, 6708 WH Wageningen (NL)
(74) Representative: Unilever Patent Group
(86) International application number: PCT/EP2023/079726
(87) International publication number: WO 2024/104743

(56) References cited:
- WO-A1-2022/089927
- WO-A2-2013/034421
- CN-A- 111 228 171
- US-A1- 2012 141 588
- DATABASE GNPD [online] MINTEL; 16 January 2020 (2020-01-16), ANONYMOUS: "Medicated Toothpaste", XP093047543, retrieved from https://www.gnpd.com/sinatra/recordpage/7192729/ Database accession no. 7192729
- DATABASE GNPD [online] MINTEL; 18 October 2022 (2022-10-18), ANONYMOUS: "Hypersensitivity Toothpaste", XP093047463, retrieved from https://www.gnpd.com/sinatra/recordpage/9981170/ Database accession no. 9981170

## Description

### Field of the Invention

The present invention relates to an oral care composition comprising calcium silicate and certain amino acids. The invention also relates to oral care compositions for use in remineralizing and/or preventing or reducing dental caries and/or reducing sensitivity of teeth of an individual by applying such compositions to the oral cavity of the individual.

### Background of the Invention

Teeth comprise dentin overlaid with an outer layer of enamel. Teeth are under constant attack from chemical and physical forces, including bacteria-derived acids and mechanical wear, resulting in demineralization and weakening of enamel and the underlying dentin. The enamel is composed hydroxyapatite crystals that create a porous surface. Dental erosion is found initially in the enamel and may proceed to the underlying dentin. There is a need to stop or at least retard the erosion of enamel to maintain good oral health. Fluoride is normally used in oral care products to combat dental caries. The resulting fluorapatite composition is harder than the original hydroxyapatite composition and more resistant to the acidic attack.

Tooth hypersensitivity is a temporary induced pain sensation that affects up to 20% of the adult population. It is associated with tooth demineralization and the loss of either enamel or cementum to expose underlying dentin. The dentin of the tooth generally contains channels, called tubules, which provide for an osmotic flow between the inner pulp region of the tooth and the outer root surfaces. The cause of tooth hypersensitivity may be related to demineralization giving rise to increased exposure of tubules and permeability of the dentine. The most common causes of demineralization of the enamel or dentine are attrition, abrasion, gingival recession and erosion. When root surfaces are exposed, dentinal tubules are also exposed.

The currently accepted theory for tooth hypersensitivity is the hydrodynamic theory, based on the belief that open exposed dentinal tubules allow fluid flow through the tubules. This flow excites the nerve endings in the dental pulp. Clinical replica of sensitive teeth viewed in a SEM (scanning electron microscopy) reveal varying numbers of open or partially occluded dentinal tubules.

Efforts have been made over the years to treat tooth hypersensitivity. One approach is to reduce the excitability of the nerve in a sensitive tooth by using "nerve-depolarising agents" comprising strontium ions, potassium salts such as potassium nitrate, potassium bicarbonate, potassium chloride and the like. These nerve-depolarising agents function by interfering with neural transduction of the pain stimulus to make the nerve less sensitive.

Another approach is to use "tubule blocking agents" that fully or partially occlude tubules such as polystyrene beads, apatite, polyacrylic acid, mineral hectorite clay and the like. These tubule blocking agents function by physically blocking the exposed ends of the dentinal tubules, thereby reducing dentinal fluid movement and reducing the irritation associated with the shear stress described by the hydrodynamic theory.

US2012/0141588A1 discloses an oral care composition including an effective amount of a basic amino acid in free or salt form; and an effective amount of calcium silicate particles. The calcium silicate particles have an average diameter of less than about 5 microns, such that they can occlude dentinal tubules of the teeth. An oral care method includes applying the composition to an oral cavity of a subject to reduce or inhibit hypersensitivity of the teeth and to achieve other benefits.

There is still a need for providing improved oral care compositions for remineralizing teeth and/or preventing or reducing dental caries and/or occluding dentinal tubules to reduce tooth sensitivity.

### Summary of the Invention

In a first aspect, the present invention is directed to an oral care composition comprising:
a) from 3 to 80% by weight of calcium silicate;
b) an uncharged polar amino acid; and
c) a charged polar amino acid;
wherein the charged polar amino acid is positively charged polar amino acids comprising lysine, arginine, histidine, ornithine or mixtures thereof.

In a second aspect, the present invention is directed to a packaged oral care product comprising the oral care composition of the first aspect of this invention.

In a third aspect, the present invention is directed to an oral care composition for use in remineralizing and/or preventing or reducing dental caries and/or reducing sensitivity of teeth of an individual comprising the step of applying the composition of any embodiment of the first aspect to the oral cavity of the individual.

All other aspects of the present invention will more readily become apparent upon considering the detailed description and examples which follow.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

The term "remineralization" in the context of the present invention means *in situ* (i.e. in the oral cavity) generation of calcium phosphate on teeth to reduce the likelihood of tooth sensitivity, tooth decay, regenerate enamel and/or improve the appearance of teeth by whitening through the generation of such new calcium phosphate. Preferably, the new calcium phosphate layers on teeth are from 10 nm to 20 microns, more preferably from 75 nm to 10 microns, and most preferably from 150 nm to 5 microns thick including all ranges subsumed therein).

The term "oral care composition" in the context of the present invention refers to a composition that is delivered to the oral surfaces. The composition may be a product which, during the normal course of usage, is not for the purpose of systemic administration or intentionally swallowed but is rather retained in the oral cavity for a time sufficient to contact substantially all of the dental surfaces and/or oral tissues for the purposes of oral activity.

Examples of the oral composition include a toothpaste or a dentifrice, a mouthwash or a mouth rinse, powder (e.g., tooth powder), lozenge, mint, cream, strip or gum (e.g., chewing gum), a film, a topical oral gel, a serum and a denture cleanser and the like. Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition of the invention preferably is used to clean the surfaces of the oral cavity. Accordingly, preferred product forms for compositions of the invention are those which are suitable for brushing and/or rinsing the surfaces of the oral cavity.

The composition of the invention is most preferably in the form of a dentifrice or a toothpaste. The term "dentifrice" in the context of the present invention refers to an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically such a composition is used in conjunction with a cleaning implement such as a toothbrush, usually by applying it to the bristles of the toothbrush and then brushing the accessible surfaces of the oral cavity. The term "toothpaste" in the context of the present invention means a paste or gel dentifrice for use with a cleaning implement such as a toothbrush. Preferably the dentifrice/toothpaste is in the form of an extrudable semi-solid such as a cream, paste or gel (or mixture thereof).

The calcium silicate suitable for use in this invention may comprise mono-calcium silicate, bi-calcium silicate, or tri-calcium silicate. In one preferred embodiment, the calcium silicate used is one which has low water solubility and is made commercially available under the name Sorbosil CA40 by PQ Corporation. In another preferred embodiment, the calcium silicate is insoluble, present as the composite material calcium oxide-silica (CaO-SiO₂), which is described, for example, in international patent application published as WO 2008/01517(Unilever). For a calcium silicate composite material, the atom ratio of calcium to silicon (Ca:Si) may be from 1:30 to 3:1. The Ca:Si ratio is preferably 1:20 to 3:1, and more preferably, from 1:10 to 3:1, and most preferably, from about 1:7 to 3:1.

In addition to calcium oxide, silica, the particles comprising the calcium silicate may comprise other components, such as metal cations, anions (such as phosphate) and the like. However, it is preferred that the particles comprise calcium oxide, silica in an amount of at least 70% by weight of the particles, more preferably at least 80%, more preferably still at least 90% and even more preferably at least 95%. Most preferably the particles consist of (or at least consist essentially of) calcium oxide, silica.

In another preferred embodiment, the calcium silicate is calcium silicate hydrate. The calcium silicate hydrate for use in the present invention comprises at least calcium oxide (CaO), silica (SiO₂) and water. Compared with conventional calcium silicate which are not hydrated, the calcium silicate hydrate comprises the water of hydration in an amount of at least 5% by weight of the calcium silicate hydrate, preferably at least 10%, more preferably at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%. "Water of hydration" for the purpose of the present invention means water chemically combined with a substance in a way that it can be removed by heating without substantially changing the chemical composition of the substance. In particular, water which could only be removed when heated above 200°C. The water loss is measured using thermo gravimetric analysis (TGA) with a Netzsch TG instrument. The TGA is conducted under an N₂ atmosphere with heating rate of 10 degree/min in the range of 30 to 900°C.

The calcium silicate hydrate preferably comprises at least 20% silica by weight of the calcium silicate hydrate, more preferably at least 30%, more preferably still at least 40% and most preferably at least 55%. The silica content is preferably no greater than 70% by weight of the calcium silicate hydrate, more preferably no greater than 65% and most preferably no greater than 60%. To provide calcium necessary for remineralization, the calcium silicate hydrate preferably comprises calcium oxide in an amount of at least 5% by weight of the calcium silicate hydrate, more preferably at least 7%, more preferably still at least 10%, even more preferably at least 12% and most preferably at least 15%. The calcium oxide content is typically no greater than 50% by weight of the calcium silicate hydrate, more preferably no greater than 40%, even more preferably no greater than 30% and most preferably no greater than 25%. The calcium silicate hydrate preferably comprises Ca and Si in an atom ratio (Ca:Si) less than 1:1, more preferably less than 1:1.2, more preferably still from 1:1.5 to 1:4 and most preferably from 1:1.7 to 1:3. In addition to calcium oxide, silica and water, the particles which comprise the calcium silicate hydrate may comprise other components, such as metal cations, anions (such as phosphate) and the like. However, it is preferred that the particles comprise CaO, SiO₂ and water in an amount of at least 70% by weight of the particles, more preferably at least 80%, more preferably still at least 90% and even more preferably at least 95%. Most preferably the particles consist of (or at least consist essentially of) CaO, SiO₂ and water.

It is preferable that the calcium silicate of the present invention is particulate which allows for maximum surface area for contact with dental tissue. Preferably the calcium silicate used in this invention has a particle size from 100 nm to 50 microns, more preferably from 500 nm to 30 microns, more preferably still from 700 nm to 20 microns and most preferably from 1 micron to 15 microns. "Particle size" in the context of the present invention means D50 particle size. The D50 particle size of a particulate material is the particle size diameter at which 50 wt% of the particles are larger in diameter and 50 wt% are smaller in diameter.

The calcium silicate may be crystalline or amorphous or mesoporous. In another preferred embodiment, the calcium silicate is at least partially crystalline. When the calcium silicate is partially crystalline, it comprises calcium (Ca) and silicon (Si) in an atom ratio (Ca:Si) from 1:3 to 3:1, more preferably from 1:1.5 to 1.5:1 and most preferably from 1:1.1 to 1.1:1. It is preferable that the calcium silicate is fully crystalline. The presence of crystallinity may be confirmed, for example, by x-ray diffraction. An example of the calcium silicate suitable for use in the present invention is commercially available under the trade name NYAD^{®} M9000 from NYCO Minerals, Inc.

The oral care composition typically comprises from 3 to 80% by weight of the calcium silicate, more preferably from 3 to 50%, even more preferably from 3 to 30% and most preferably from 3 to 20%, based on total weight of the oral care composition and including all ranges subsumed therein.

Amino acids are organic molecules that consist of a basic amino group (-NH₂) and an acidic carboxyl group (-COOH) and an organic side chain that is unique to each amino acid. It is known so far that there are 20 amino acids in the standard genetic code and the others (e.g., selenocysteine, pyrrolysine, ornithine) are found in a few bacteria. The 20 amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine.

The amino acids can be divided into two groups: nonpolar amino acids and polar amino acids. Polar amino acids have hydrophilic side chains, which can be further divided into three groups: uncharged (neutral) polar amino acids, positively charged polar amino acids and negatively charged polar amino acids.

The uncharged polar amino acid suitable for use in this invention includes, for example, serine, tyrosine, threonine, cysteine, asparagine, glutamine or mixtures thereof, preferably serine, tyrosine, cysteine or mixtures thereof. Tyrosine is particularly preferred.

The composition preferably comprises the uncharged polar amino acid from 0.01 to 15% by weight of the composition, more preferably from 0.1 to 10%, even more preferably from 0.5 to 8% and most preferably from 1 to 5%, based on total weight of the composition and including all ranges subsumed therein.

The charged polar amino acid suitable for use in the present invention is positively charged polar amino acid, which comprises lysine, arginine, histidine, ornithine or mixtures thereof, preferably the positively charged polar amino acid comprises or is lysine.

It is especially preferred that the uncharged polar amino acid is tyrosine and the charged polar amino acid is lysine.

It is also preferred that the charged polar amino acid suitable for use in the present invention is a combination of positively charged polar amino acids and negatively charged polar amino acids. Suitable positively charged polar amino acid comprises lysine, arginine, histidine, ornithine or mixtures thereof, preferably the positively charged polar amino acid comprises or is lysine. Suitable negatively charged polar amino acids comprises aspartic acid, glutamic acid or mixtures thereof, preferably the negatively charged polar amino acid comprises or is glutamic acid. The combination preferably comprises the positively charged polar amino acid and the negatively charged polar amino acid in a weight ratio from 1:20 to 10:1, more preferably from 1:10 to 5:1.

Preferably the charged polar amino acid is present in the composition in an amount of from 0.01 to 15% by weight of the composition, more preferably from 0.1 to 10%, even more preferably from 0.5 to 8% and most preferably from 1 to 5%, based on total weight of the composition and including all ranges subsumed therein.

The weight ratio of the uncharged polar amino acid to the charged polar amino acid in the composition is preferably from 1:15 to 15:1, more preferably from 1:10 to 10:1, and most preferably from 1:5 to 5:1.

The total amount of uncharged polar amino acid and charged polar amino acid in the composition is preferably from 0.1 to 20% by weight of the composition, more preferably from 1 to 15%, even more preferably from 2 to 10% and most preferably from 3 to 8%, based on total weight of the oral care composition and including all ranges subsumed therein.

The composition of the present invention may comprise other amino acids in addition to the uncharged polar amino acid and the charged polar amino acid. Preferably, the composition is substantially free of other amino acids. The term "substantially free of" in the context of the present invention means less than 0.005%, preferably less than 0.001%, more preferably less than 0.0005%, even more preferably from 0 to 0.0005% by weight, based on total weight of the composition, including all ranges subsumed therein. Preferably, the composition of the present invention does not comprise other amino acids in addition to the uncharged polar amino acid and the charged polar amino acid which are included in the composition.

Preferably, the amount of calcium silicate and the total amount of uncharged polar amino acid and charged polar amino acid are present in the composition at a weight ratio from 1:10 to 20:1, preferably from 1:5 to 15:1, more preferably from 1:3 to 13:1, even more preferably from 1:1 to 10:1, including all ratios subsumed therein.

The composition may comprise a phosphate source. The phosphate source is able to provide phosphate ions to react with the calcium silicate to produce a calcium phosphate *in situ* reaction product that is a precursor for hydroxyapatite formation. Preferably the phosphate source dissolves in water to give a phosphate ion concentration of at least 0.1 moles per liter at room temperature and atmospheric pressure. Ilustrative examples of the types of phosphate source suitable for use in this invention include trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate, mixtures thereof or the like.

The phosphate source may be present in the range of from 0.1 to 30%, preferably from 0.5 to 20%, more preferably from 1 to 15% by weight of the composition, based on total weight of the composition and including all ranges subsumed therein.

The calcium silicate and the phosphate source may be present in a weight ratio from 1:10 to 30:1, preferably from 1:5 to 20:1, more preferably from 1:3 to 15:1.

Preferably, the composition of the invention is non-aqueous. It may improve the stability of many materials which are physically or chemically incompatible with the aqueous environments. For example, presence of calcium silicate and a phosphate source in a monophase aqueous composition can lead to premature reaction of the calcium and phosphate and instability of the product. By "non-aqueous" it is generally meant that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" generally means that water is present in an amount no greater than 5%, more preferably no greater than 3% by weight based on the total weight of the composition.

Typically, the composition has a pH from 5.5 to 10.5, more preferably from 6.0 to 10, and most preferably from 6.5 to 9.5. The pH of composition may be measured when 5 parts by weight of the composition is uniformly dispersed and/or dissolved in 20 parts by weight pure water at 25°C. In particular, the pH may be measured by manually mixing 5 g composition with 20 mL water for 30 s, then immediately testing the pH with indicator or a pH meter, or the pH of composition may be measured directly with a pH meter.

The composition of the present invention may also comprise a physiologically acceptable carrier. The carrier preferably comprises at least surfactant, thickener, humectant or a combination thereof.

Preferably the composition comprises a surfactant. Preferably the composition comprises at least 0.01% surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine, sodium methyl cocoyl taurate or mixtures thereof.

Thickener may also be used in this invention. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In a preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another preferred embodiment, the thickener is xanthan gum.

In another especially preferred embodiment, the thickener is carrageenan. Suitable carrageenan includes iota and kappa carrageenan. Preferably the iota carrageenan and the kappa carrageenan are present in a weight ratio of from 1:2 to 2:1. Preferably the carrageenan present in the composition of the invention consists from 33 to 66% by weight of iota carrageenan and 33 to 66% by weight of kappa carrageenan. The total level of carrageenan is preferably from 0.05 to 1% by weight of the composition, more preferably 0.08 to 0.5%, and most preferably 0.08 to 0.25% by weight of the composition. Iota carrageenan or kappa carrageenan is commercially available, for example, from CP Kelco.

Thickener typically makes up from 0.01 to 10%, more preferably from 0.1 to 9%, and most preferably, from 0.1 to 5% by weight of the composition, based on total weight of the composition and including all ranges subsumed therein.

Suitable humectants are preferably used in the composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the composition. More preferably, the carrier humectant makes up from 25 to 80%, and most preferably, from 30 to 60% by weight of the composition, based on total weight of the composition and including all ranges subsumed therein.

The composition may comprise a fluoride source. Preferred fluoride source includes sodium fluoride, stannous fluoride, potassium fluoride, sodium monofluorophosphate, sodium fluorosilicate, ammonium fluorosilicate, amine fluoride, ammonium fluoride or mixtures thereof. Preferably, the fluoride source is stannous fluoride, sodium fluoride, sodium monofluorophosphate or mixtures thereof. Sodium monofluorophosphate is particularly preferred. The fluoride source may be present at a level from 0.01 to 10%, more preferably from 0.03 to 5% and most preferably from 0.1 to 2% by weight of the composition, based on total weight of the composition and including all ranges subsumed therein.

The composition may comprise particulate abrasive materials such as silicas, aluminas, calcium carbonates, dicacliumphosphates, calcium pyrophosphates, hydroxyapatites, trimetaphosphates, insoluble hexametaphosphates and so on, including agglomerated particulate abrasive materials. The abrasives may be present in the range of from 0.01 to 60%, more preferably from 0.1 to 30%, and most preferably from 1 to 15% by weight of the composition, based on total weight of the composition and including all ranges subsumed therein.

Preferably the composition, particularly a toothpaste, comprises a silica based abrasive. The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 to 1.47, preferably 1.435 to 1.445, preferably having a weight mean particle size of between 5 and 15 mm, a BET (nitrogen) surface area of between 10 and 100 m²/g and an oil absorption of 70 to 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Evonik, Sorbosil AC 77 ex PQ Corporation (having an R.I. of approximately 1.440). The amount of these silicas in the composition generally ranges from 5 to 60% by weight, usually 5 to 20% by weight.

The composition may comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

The composition may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the total level of pigment in the composition is from 0.01 to 3% by weight of the composition, more preferably from 0.02 to 2%.

The composition may comprise water-soluble or sparingly water-soluble sources of metal salts Preferred are zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate; also preferred are stannous ions such as stannous fluoride and stannous chloride.

The composition may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof;

Mixtures of any of the above described materials may also be used.

The composition of the present invention may contain a variety of other ingredients which are common in the art to enhance physical properties and performance in addition to the ingredients specified above, such as:
antimicrobial agents, e.g. chlorhexidine, sanguinarine extract, metronidazole, quaternary ammonium compounds, such as cetylpyridinium chloride; cetylpyridium chloride clay complex bis-guanides, such as chlorhexidine digluconate, hexetidine, octenidine, alexidine; and
halogenated bisphenolic compounds, such as 2,2' methylenebis-(4-chloro-6-bromophenol); anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers,
organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
hyaluronic acid;
colouring agents;
pH-adjusting agents;
sweetening agents;
polymeric compounds which can enhance the delivery of active ingredients such as antimicrobial agents can also be included;
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

Such ingredients typically and collectively make up less than 20% by weight of the composition, and preferably, from 0.0 to 15% by weight, and most preferably, from 0.01 to 12% by weight of the composition, including all ranges subsumed therein.

The present invention is directed to an oral care composition for use in remineralizing and/or preventing or reducing dental caries and/or reducing sensitivity of teeth of an individual. Additionally or alternatively, the present invention is directed to use of an oral care composition for remineralizing and/or preventing or reducing dental caries and/or reducing sensitivity of teeth of an individual. Additionally or alternatively, the present invention is directed to use of an oral care composition in the manufacture of a medicament in remineralizing and/or preventing or reducing dental caries and/or reducing sensitivity of teeth of an individual.

Additionally, application of the composition of the present invention to the oral cavity of an individual may provide one or more of the following benefits: reduce plaque accumulation; inhibit microbial biofilm formation in the oral cavity; reduce or inhibit gingivitis; promote healing of sores or cuts in the mouth; reduce levels of acid producing bacteria; increase relative levels of non-cariogenic and/or non-plaque forming bacterial; reduce, repair or inhibit pre-carious lesions of the enamel; treat, relieve or reduce dry mouth; clean the teeth and oral cavity; reduce erosion; whiten teeth; reduce tartar build-up; and/or promote systemic health, including cardiovascular health, e.g., by reducing potential for systemic infection via the oral tissues. The disclosure further provides compositions for use in any of the above methods.

The composition can be effective even when used in an individual's daily oral hygiene routine. For example, the composition may be brushed onto the teeth. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day.

The following examples are provided to facilitate an understanding of the present invention. The examples are not provided to limit the scope of the claims.

### Examples

### Example 1

Compositions were prepared as shown in table 1. All ingredients are expressed by weight percent of the total formulation.

**TABLE 1**

| **Ingredient** | **Samples** | | | |
|---|---|---|---|---|
| | **A** | **B** | **C** | **1** |
| Glycerin | To 100 | To 100 | To 100 | To 100 |
| Calcium silicate^{a} | 15 | 15 | 15 | 15 |
| Trisodium phosphate | 3.8 | 3.8 | 3.8 | 3.8 |
| Monosodium phosphate | 3.2 | 3.2 | 3.2 | 3.2 |
| PEG 400 | 2.5 | 2.5 | 2.5 | 2.5 |
| Tyrosine | - | 4 | - | 2.21 |
| Lysine | - | - | 4 | 1.79 |
| Sodium lauryl sulfate | 2 | 2 | 2 | 2 |
| Abrasive silica | 2 | 2 | 2 | 2 |
| Sodium monofluorophospahte | 1.11 | 1.11 | 1.11 | 1.11 |
| Sodium saccharin | 0.22 | 0.22 | 0.22 | 0.22 |
| Kappa carrageenan | 0.08 | 0.08 | 0.08 | 0.08 |
| Iota carrageenan | 0.08 | 0.08 | 0.08 | 0.08 |
| Perfume | 0.7 | 0.7 | 0.7 | 0.7 |

| | | | | |
|---|---|---|---|---|
| a. Commercially available calcium silicate under the trade name Sorbosil CA40 from P.Q. Corporation | | | | |

### Evaluation of surface microhardness (SMH) reduction of tooth enamels

Fresh toothpaste slurries were prepared by mixing 4 g toothpaste sample with 8 mL de-ionised (DI) water for 40 seconds and used immediately.

The enamel surface microhardness (SMH) was measured by a microhardness tester (Struers Durascan) using a Knoop indenter at 50gf load for 10 seconds. Five indentations were made per test point for each enamel block in different regions to avoid residual stress. To evaluate the microhardness of tooth enamel, well-polished human enamel blocks were divided into four groups (n=5 per group) with similar baseline values of SMH. The SMH of the human enamel blocks were measured and recorded as SMH_{baseline}. The human enamel blocks were soaked in freshly prepared toothpaste slurry for 3 minutes and then placed in demineralization solution (Demin solution) for 4 hours at 37°C with mild shaking, followed by rinsing with water. After that, the enamel blocks were soaked in remineralization solution (Remin solution) for 4 hours at 37°C with mild shaking. The whole treatment within one day was called one-day treatment cycle. The treatment was repeated for 7 days and the SMH of the enamel blocks were measured and recorded as SMH_{treated}.

Demin solution consisted of 2 mM CaCl₂, 2 mM KH₂PO₄, 75 mM acetic acid, 0.1 mM Tris buffer, pH adjusted to 4.6 using 1 M KOH.

Remin solution consisted of 1.5 mM CaCl₂, 0.9 mM KH₂PO₄, 150 mM KCI, and 20 mM HEPES adjusted to pH 7.0 with NaOH.

The surface microhardness (SMH) reduction of tooth enamels was calculated as: $SMH Reduction \left(%\right) = \left({SMH}_{treated}-{SMH}_{baseline}\right) \times 100 / {SMH}_{baseline}$

The results are shown in table 2.

**TABLE 2**

| **SMH Reduction/%** | **Samples** | | | |
|---|---|---|---|---|
| | **A** | **B** | **C** | **1** |
| | -21.25 | -20.0 | -21.4 | -18.25 |

It can be seen in table 2 that the enamel blocks treated with sample 1 was more resistant to acid attack, indicating better enamel protection from acid erosion.

### Evaluation of blockage efficacy of dentinal tubules

Fresh toothpaste slurries were prepared by mixing 4 g toothpaste sample with 8 mL de-ionised (DI) water for 40 seconds and used immediately.

Human dentine discs were eroded by 6% citric acid for 2 minutes, then they were treated with different slurries via brushing following the same protocol. Eight human dentine discs were separated into four groups (n=2). The dentine discs were brushed with the slurry under a tooth brushing machine equipped with toothbrushes. The load of the tooth brushing was 170 g +/-5 g and the automatic brushing operated at a speed of 150 rpm. After brushing for 1 minute, the dentine discs were soaked in slurry for 1 minute. Then the dentine discs were placed in 50 mL DI water and agitated on a flatbed shaker at 150 rpm for 10 strokes. The discs were then soaked in simulated oral fluid (SOF) for at least 6 hours under the condition of a shaking water bath at 37°C and 60.0 rpm. After that, the dentine discs were brushed with the slurry by machine using the same procedure as in the first step. The brushing was repeated twice for one day, then the dentine discs were kept in SOF overnight (>12 hours) in a shaking water bath at 37°C to mimic oral environment. The dentine samples were characterized by scanning electron microscopy (SEM, Hitachi S-4800, Japan) after one day of brushing.

Simulated oral fluid was made by combining the ingredients in table 3:

**TABLE 3**

| **Ingredient** | **Amount/g** |
|---|---|
| NaCl | 16.07 |
| NaHCO₃ | 0.7 |
| KCI | 0.448 |
| K₂HPO₄*3H₂O | 3.27 |
| MgCl₂*6H₂O | 0.0622 |
| 1M HCl | 40 mL |
| CaCl₂ | 0.1998 |
| Na₂SO₄ | 0.1434 |
| Buffer | Adjust pH to 7.0 |
| Water | Balance to 2 L |

Regardless of the original shape of the dentine discs, a square (with a size of 4mm x 4mm) is selected and one image is captured under 30x magnification. Within this square, five spots (each with a size of 150 µm x 150 µm, one in the middle, and one in every corner) are selected and observed under 1000x magnification. The blockage of tubules is accessed following the standards described in Table 4. The measurement is carried out for the two dentine discs of each test group.

**TABLE 4**

| **Score** | **Tubules Blockage** |
|---|---|
| 0 | All dentinal tubules are open. |
| 1 | <20% of dentinal tubules are fully blocked. |
| 2 | 20 to 50% of dentinal tubules are fully blocked. |
| 3 | 50 to 80% of dentinal tubules are fully blocked. |
| 4 | 80-100% of dentinal tubules are fully blocked. |
| 5 | All dentinal tubules are fully blocked. |

SEM images of the dentine discs were taken after one day of brushing. The images were analyzed and scored. The results are reported in table 5.

**TABLE 5**

| **Tubules Blockage Score** | **Samples** | | | |
|---|---|---|---|---|
| | A | B | C | 1 |
| One day of brushing | 3.1 | 4.2 | 3.8 | 5 |

Sample 1 in accordance with the present invention provided better tubule blockage efficacy than comparative samples A, B and C.

## Claims

1. An oral care composition comprising:
a) from 3 to 80% by weight of calcium silicate;
b) an uncharged polar amino acid; and
c) a charged polar amino acid;
wherein the charged polar amino acid is positively charged polar amino acids comprising lysine, arginine, histidine, ornithine or mixtures thereof.

2. The oral care composition according to claim 1, wherein the uncharged polar amino acid comprises serine, tyrosine, threonine, cysteine, asparagine, glutamine or mixtures thereof, preferably serine, tyrosine, cysteine or mixtures thereof, more preferably tyrosine.

3. The oral care composition according to claim 1 or claim 2, wherein the positively charged polar amino acid is lysine.

4. The oral care composition according to any of the preceding claims, wherein the uncharged polar amino acid and the charged polar amino acid are present in the composition in a weight ratio from 1:15 to 15:1, preferably from 1:10 to 10:1.

5. The oral care composition according to any of the preceding claims, wherein the uncharged polar amino acid is tyrosine and the charged polar amino acid is lysine.

6. The oral care composition according to any of the preceding claims, wherein total amount of uncharged polar amino acid and charged polar amino acid in the composition ranges from 0.1 to 20%, preferably from 1 to 15%.

7. The oral care composition according to any of the preceding claims, wherein the calcium silicate comprises Ca and Si in an atom ratio of from 1:30 to 3:1, preferably from 1:20 to 3:1.

8. The oral care composition according to any of the preceding claims, wherein the calcium silicate is present in an amount of from 3 to 50% by weight of the composition, preferably from 3 to 30%.

9. The oral care composition according to any of the preceding claims, wherein the amount of calcium silicate and the total amount of uncharged polar amino acid and charged polar amino acid are present at a weight ratio from 1:10 to 20:1, preferably from 1:5 to 15:1.

10. The oral care composition according to any of the preceding claims, wherein the composition additionally comprises a phosphate source.

11. The oral care composition according to any of the preceding claims, wherein the composition is a non-aqueous composition.

12. An oral care composition according to any of the preceding claims for use in remineralizing and/or preventing or reducing dental caries and/or reducing sensitivity of teeth of an individual comprising the step of applying the composition to the oral cavity of the individual.

## Patentansprüche

1. Mundpflegezusammensetzung, die umfasst:
a) 3 bis 80 Gewichtsprozent Calciumsilikat;
b) eine ungeladene polare Aminosäure; und
c) eine geladene polare Aminosäure;
wobei die geladene polare Aminosäure positiv geladene polare Aminosäuren darstellt, umfassend Lysin, Arginin, Histidin, Ornithin oder Mischungen davon.

2. Mundpflegezusammensetzung nach Anspruch 1, wobei die ungeladene polare Aminosäure Serin, Tyrosin, Threonin, Cystein, Asparagin, Glutamin oder Mischungen davon umfasst, vorzugsweise Serin, Tyrosin, Cystein oder Mischungen davon, bevorzugter Tyrosin, umfasst.

3. Mundpflegezusammensetzung nach Anspruch 1 oder Anspruch 2, wobei die positiv geladene polare Aminosäure Lysin ist.

4. Mundpflegezusammensetzung nach einem der vorhergehenden Ansprüche, wobei die ungeladene polare Aminosäure und die geladene polare Aminosäure in der Zusammensetzung in einem Gewichtsverhältnis von 1:15 bis 15:1 vorliegen, vorzugsweise von 1:10 bis 10:1.

5. Mundpflegezusammensetzung nach einem der vorhergehenden Ansprüche, wobei die ungeladene polare Aminosäure Tyrosin und die geladene polare Aminosäure Lysin ist.

6. Mundpflegezusammensetzung nach einem der vorhergehenden Ansprüche, wobei die Gesamtmenge der ungeladenen polaren Aminosäure und der geladenen polaren Aminosäure in der Zusammensetzung im Bereich von 0,1 bis 20% liegt, vorzugsweise von 1 bis 15%.

7. Mundpflegezusammensetzung nach einem der vorhergehenden Ansprüche, wobei das Calciumsilikat Ca und Si in einem Atomverhältnis von 1:30 bis 3:1 umfasst, vorzugsweise von 1:20 bis 3:1.

8. Mundpflegezusammensetzung nach einem der vorhergehenden Ansprüche, wobei das Calciumsilikat in einer Menge von 3 bis 50 Gew.-% vorliegt, vorzugsweise von 3 bis 30% der Zusammensetzung.

9. Mundpflegezusammensetzung nach einem der vorhergehenden Ansprüche, wobei die Menge an Calciumsilikat und die Gesamtmenge an ungeladener polarer Aminosäure und geladener polarer Aminosäure in einem Gewichtsverhältnis von 1:10 bis 20:1 vorliegen, vorzugsweise von 1:5 bis 15:1.

10. Mundpflegezusammensetzung nach einem der vorhergehenden Ansprüche, wobei die Zusammensetzung zusätzlich eine Phosphatquelle umfasst.

11. Mundpflegezusammensetzung nach einem der vorhergehenden Ansprüche, wobei die Zusammensetzung eine nicht-wässrige Zusammensetzung ist.

12. Mundpflegezusammensetzung nach einem der vorhergehenden Ansprüche zur Verwendung bei der Remineralisierung und/oder der Vorbeugung oder der Verringerung von Zahnkaries und/oder der Verringerung der Empfindlichkeit der Zähne einer Person, umfassend den Schritt des Aufbringens der Zusammensetzung auf die Mundhöhle der Person.

## Revendications

1. Composition de soins bucco-dentaires comprenant:
a) de 3 à 80 % en poids de silicate de calcium;
b) un acide aminé polaire non chargé; et
c) un acide aminé polaire chargé;
où l'acide aminé polaire chargé est un acide aminé polaire chargé positivement comprenant la lysine, l'arginine, l'histidine, l'ornithine ou des mélanges de celles-ci.

2. Composition de soins bucco-dentaires selon la revendication 1, où l'acide aminé polaire non chargé comprend la sérine, la tyrosine, la thréonine, la cystéine, l'asparagine, la glutamine ou des mélanges de celles-ci, de préférence la sérine, la tyrosine, la cystéine ou des mélanges de celles-ci, de préférence encore la tyrosine.

3. Composition de soins bucco-dentaires selon la revendication 1 ou la revendication 2, où l'acide aminé polaire chargé positivement est la lysine.

4. Composition de soins bucco-dentaires selon l'une quelconque des revendications précédentes, où l'acide aminé polaire non chargé et l'acide aminé polaire chargé sont présents dans la composition dans un rapport pondéral de 1:15 à 15:1, de préférence de 1:10 à 10:1.

5. Composition de soins bucco-dentaires selon l'une quelconque des revendications précédentes, où l'acide aminé polaire non chargé est la tyrosine et l'acide aminé polaire chargé est la lysine.

6. Composition de soins bucco-dentaires selon l'une quelconque des revendications précédentes, où la quantité totale d'acide aminé polaire non chargé et d'acide aminé polaire chargé dans la composition va de 0,1 à 20 %, de préférence de 1 à 15 %.

7. Composition de soins bucco-dentaires selon l'une quelconque des revendications précédentes, où le silicate de calcium comprend Ca et Si dans un rapport atomique de 1:30 à 3:1, de préférence de 1:20 à 3:1.

8. Composition de soins bucco-dentaires selon l'une quelconque des revendications précédentes, où le silicate de calcium est présent en une quantité de 3 à 50 % en poids de la composition, de préférence de 3 à 30 %.

9. Composition de soins bucco-dentaires selon l'une quelconque des revendications précédentes, où la quantité de silicate de calcium et la quantité totale d'acide aminé polaire non chargé et d'acide aminé polaire chargé sont présentes à un rapport pondéral de 1:10 à 20:1, de préférence de 1:5 à 15:1.

10. Composition de soins bucco-dentaires selon l'une quelconque des revendications précédentes, où la composition comprend en outre une source de phosphate.

11. Composition de soins bucco-dentaires selon l'une quelconque des revendications précédentes, où la composition est une composition non aqueuse.

12. Composition de soin bucco-dentaires selon l'une quelconque des revendications précédentes destinée à être utilisée dans la reminéralisation et/ou la prévention ou la réduction des caries dentaires et/ou la réduction de la sensibilité des dents d'un individu comprenant l'étape d'application de la composition à la cavité buccale de l'individu.
